(19) **Europäisches Patentamt European Patent Office Office européen des brevets**

(11) **EP 4 324 488 A1**

(12) # EUROPEAN PATENT APPLICATION

(43) Date of publication:
**21.02.2024   Bulletin 2024/08**

(21) Application number: **22191064.9**

(22) Date of filing: **18.08.2022**

(51) International Patent Classification (IPC):
**A61L 27/04** (2006.01)      **A61L 27/42** (2006.01)
**A61L 27/58** (2006.01)

(52) Cooperative Patent Classification (CPC):
**A61L 27/425; A61L 27/047; A61L 27/427;
A61L 27/58;** A61L 2430/02

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **ETH Zurich
8092 Zurich (CH)**

(72) Inventors:
• **LÖFFLER, Jörg**
  **8606 Greifensee (CH)**
• **FERGUSON, Stephen**
  **8340 Hinwil (CH)**
• **BERGER, Leopold**
  **8006 Zürich (CH)**
• **HELGASON, Benedikt**
  **8046 Zürich (CH)**
• **PERSSON, Karin**
  **75679 Uppsala (SE)**
• **DELLER, Robin**
  **8049 Zürich (CH)**

(54)    **FIBER REINFORCED BONE CEMENT**

(57)    The present invention relates to bone cement composites comprising at least one cement base material and biodegradable metallic fibers made of a metal alloy, said fibers comprising at least three chemical elements in the alloy, wherein at least one of these elements is selected from the group consisting of magnesium and calcium. The fibers are amorphous in a volume fraction of more than 75%, with the remaining part of the fibers being of crystalline atomic structure.

Figure 6A

**EP 4 324 488 A1**

**Description**

The present invention relates to a bone cement composite comprising at least one cement base material and biodegradable metallic fibers made of a metal alloy.

Amorphous metals in general, but also biodegradable amorphous metals, are well known in the art. Furthermore, fiber-reinforced bone cements are known as well.

US2014/0007986A1 describes a composite including a matrix material having an intrinsic strain-to-failure rating in tension and a reinforcing material embedded in the bulk material. However, said composite requires pre-tensioning of the fibers and does not mention biodegradability or a cementitious matrix material.

CN106273680A discloses a non-biodegradable cobalt-based amorphous alloy fiber composite for sensor applications. Furthermore, US5543187A describes a multilaminate composite material made of an amorphous metallic material and a ceramic material.

Composites of biodegradable bone cement with biodegradable fibers of various biodegradable materials are known. I. Lodoso-Torrecilla, J.J.J.P. van den Beucken, J.A. Jansen (Calcium phosphate cements: Optimization toward biodegradability. Acta Biomater. 2021;119:1-12) disclose calcium phosphate cements which are reinforced with fibers made of polylactic acid (PLA), poly(lactic-co-glycolic acid) (PLGA), poly(vinyl alcohol)(PVA), gelatin, chitosan, glass or carbon.

R. Krüger, J.M. Seitz, A. Ewald, F.W. Bach, J. Groll (Strong and tough magnesium wire reinforced phosphate cement composites for load-bearing bone replacement. J Mech Behav Biomed Mater. 2013;20(C):36-44) disclose a composite of a biodegradable magnesium calcium phosphate cement with reinforcement of uniaxially orientated fibers of a polycrystalline magnesium alloy. The mechanical properties of said composite are not sufficient. It was reported that failure of the composite occurs in vicinity of the wire-matrix interface. Due to the low fiber-cement interface strength, cracks driven by shear stresses propagate parallel to the fibers when loading the construct in bending mode. Furthermore, due to the reported long and rather thick fibers, combined with their uniaxial orientation, the reported composite does not allow an intra-surgical application. From the reported data it is safe to assume that also a composite of randomly orientated fibers of the same kind would not provide a significant mechanical improvement to plain bone cement without fibers. This assessment is based on the reported weak fiber-cement interface strength and the limited mechanical strength of the used fiber material.

Currently available bone cements show excellent biocompatibility and degradability. However, their tensile, shear and bending strength is rather low and as major issue, they are exceptionally brittle, which diminishes their applicability in load-bearing applications.

The object of the present invention was therefore, to provide a bone cement composite with enhanced strength and ductility while at the same time being biocompatible.

The problem is solved by the subject-matter of independent claim 1. Further preferred embodiments are subject of dependent claims 2 to 15.

The biocompatible bone cement composite according to the present invention comprises at least one cement base material and metallic fibers made of a metal alloy, said fibers comprising at least three chemical elements in the alloy, wherein at least one of these elements is selected from the group consisting of magnesium and calcium, wherein the fibers are amorphous in a volume fraction of more than 75%, with the remaining part of the fibers being of crystalline atomic structure.

The biocompatible fiber-reinforced bone cement composite comprises fibers of a metallic alloy which is amorphous in a volume fraction of more than 75%. This highly amorphous, i.e., disordered, atomic-scale structure of the alloy results in a two to fourfold increased mechanical strength of the here employed fiber material compared to respective crystalline counterparts. The fibers can be randomly oriented, partly oriented, or fully uniaxially oriented within the bone cement matrix. For example, it is possible to orient fibers within the bone cement matrix according to expected stress trajectories of the later loading.

The fibers are embedded in a cement base material resulting in a fiber-reinforced bone cement composite having significantly enhanced strength and/or ductility, by not compromising biocompatibility and degradability the same time. This allows for an application in load-bearing applications. Furthermore, the bone cement composite according to the present invention can be osteoconductive and osteoinductive by the degradation of the fibers. In addition, the bone cement according to the present invention has an excellent injectability, which is a clinically important issue.

The fibers contained in the bone cement composite comprise at least three chemical elements as alloying elements, wherein at least one of these elements is selected from the group consisting of magnesium and calcium.

Preferably, the bone cement composite according to the present invention comprises fibers which are amorphous in a volume fraction of more than 90%, or more preferable in a volume fraction of 95%, or particularly preferable in a volume fraction of more than 98%, with the remaining part of the fibers being of crystalline atomic structure. A high amorphous volume fraction has a direct impact on the strength and ductility of the cement of the invention. The amorphous structure of the fibers contained in the bone cement composite according to the present invention results in multiple times higher fiber-strength compared to crystalline alloys or polymers, which allows for large reduction of fiber length and diameter at the same strengthening effect. Shorter, and thinner fibers add to malleability and injectability of the bone cement.

One embodiment of the present invention relates to a bone cement composite comprising uncoated fibers. It was found out that uncoated fibers can react with the setting solution resulting in gas formation. The gas forms pores in the final bone cement. These pores have an effect on the mechanical properties. Maximum flexural strength and flexural modulus are both decreased, though the ductility is still present. On the other hand, the pore formation results in an increased volume and expansion during the setting process. Bone cements comprising uncoated fibers are of particular interest for applications where bone cements are already used, and no or very limited mechanical loads are present. Pores are beneficial and even necessary for fast ingrowth of bone tissue, enhancing the cement-bone interface. Moreover, the fast ingrowth of bone can further stabilize and strengthen an implant. Another advantage is that they can be used in filling defects with complicated geometries. Due to the volume increase after the injection, the cement fills voids and gaps that are not accessible otherwise.

In another embodiment of the present invention the fibers comprised in the bone cement composite are at least partly, preferably completely, coated with a biodegradable degradation-protective layer. Such bone cements are particularly interesting for load-bearing application, since they have outstanding mechanical properties. Said protective layer prevents a chemical reaction between the fibers and the setting solution during setting of the cement.

Preferably said protective layer comprises salts, in particular phosphate salts or fluoride salts, oxides, biodegradable polymers, or small biocompatible organic molecules.

Coatings with phosphate salts can be obtained for example by dipping the fibers into a $(NH_4)_2HPO_4$ solution or by another form of phosphate conversion coating and subsequent drying. Said coatings have a dense structure and excellent adhesion to the cement base material. Furthermore, they show good biocompatibility.

Coatings with fluoride salts can be obtained for example by hydrofluoric acid immersion treatment. Said coating significantly improves the corrosion resistance of the fibers during setting of the cement while also meeting the requirements of self-degradability and biocompatibility.

Biodegradable polymers are preferably selected from the group consisting of polycaprolactone (PCL), poly(L-lactic acid) (PLLA), poly(DL-lactide-co-glycolide) (PLGA), poly(ether imide)(PEI), polyethyleneglycol(PEG), polyhydroxybutyrate (PHB), alginate, and chitosan or mixtures thereof.

Small biocompatible organic molecules are preferably selected from the group consisting of glucose, mannose, fructose, sucrose, lactose, maltose and trehalose.

Such coatings can be obtained for example by deposition coating, plasma electrolytic oxidation coating, plasma electrolytic anodization coating, anodization coating, a fluoric conversion coating, $Mg(OH)_2$ coating, calcium phosphate conversion coating, phosphate conversion coating, hydroxyapatite coating, organic coating, biodegradable polymer coating, and sol-gel coating.

Preferably, the bone cement composite according to the present invention comprises fibers that comprise at least 45 atomic percent (at%) calcium, thus are made of a calcium-rich material. Most preferably, the fibers contained in the bone cement composite according to the present invention comprises between 45 at% to 80 at% of calcium, between 5 at% to 30 at% of magnesium and between 2 at% and 35 at% of zinc. Magnesium, zinc and calcium are essential nutrients in the human body. Therefore, such fibers have a high biocompatibility. Additionally, in contact with water or body fluids the alloy of such fibers starts to slowly degrade with the degradation products being resorbed by the human body. Especially preferred are fibers consisting of alloys selected from the group consisting of $Ca_{57.5}Mg_{15}Zn_{27.5}$, $Ca_{55}Mg_{17.5}Zn_{27.5}$, $Ca_{52.5}Mg_{20}Zn_{27.5}$, $Ca_{52.5}Mg_{17.5}Zn_{30}$, $Ca_{52.5}Mg_{22.5}Zn_{25}$ and $Ca_{50}Mg_{20}Zn_{30}$, with the subscripted numbers giving the relative amounts of the respective element in atomic percent.

Preferably, the fibers contained in the bone cement composite according to the present invention comprises at least 55 at% magnesium, i.e., are made of a magnesium-rich material. Despite of the high amount of magnesium, the bone cement composite according to the present invention does not show any crack propagation parallel to the fibers or a delamination process. Instead, the fibers break when their mechanical limit is reached in-plane with the cement matrix, which indicates an exceptionally strong fiber-cement interface.

Especially good results are obtained with a bone cement composite, wherein the fibers comprise between 55 at% to 80 at% of magnesium, between 15 at% to 40 at% of zinc and between 1 at% and 10 at% of calcium, preferably between 60 at% to 75 at% of magnesium, between 20 at% to 35 at% of zinc and between 2 at% and 7 at% of calcium. The fibers may additionally contain up to 5 at% of one or more elements selected from the group consisting of copper, nickel, zinc, palladium, and rare-earth elements.

In one embodiment of the present invention the fibers consist of 55 at% to 80 at% of magnesium, between 15 at% to 40 at% of zinc and between 1 at% and 10 at% of calcium, preferably between 60 at% to 75 at% of magnesium, between 20 at% to 35 at% of zinc and between 2 at% and 7 at% of calcium. Such fibers have an excellent corrosion resistance resulting in less hydrogen bubbles during setting of the cement or later during degradation *in vivo*. Especially preferred are fibers consisting of alloys selected from the group consisting of $Mg_{60}Zn_{35}Ca_5$, $Mg_{66}Zn_{30}Ca_4$, $Mg_{69}Zn_{26}Ca_5$,

$Mg_{67}Zn_{28}Ca_5$, $Mg_{69}Zn_{27}Ca_4$, $Mg_{69}Zn_{28}Ca_3$ and $Mg_{72}Zn_{24}Ca_4$, with the subscripted numbers giving the relative amounts of the respective element in atomic percent.

In one aspect of the present invention, the fibers comprise least 55 at% magnesium or at least 45 at% calcium, and one or more elements selected from the group consisting of copper, nickel, zinc, palladium, and rare-earth elements, preferably magnesium and calcium and one or more elements selected from the group consisting of copper, nickel, zinc and palladium. Preferred rare-earth elements are yttrium and ytterbium.

The bone cement composite according to the present invention comprises fibers having a diameter which is smaller than 150 $\mu$m, preferably smaller than 100 $\mu$m and most preferably smaller than 50 $\mu$m. A smaller fiber diameter simultaneously allows for a decrease of the fiber length without compromising the composite material's overall strength. And shorter fibers facilitate intra-surgical application by enhancing malleability and injectability of the composite during its setting and intra-surgical application phase. Therefore, preferably, fibers are as short as possible without compromising mechanical parameters of the resulting composite material. The resulting excellent malleability of the bone cement composite according to the present invention allows for the cement to better adapt to a defect's site and shape, and short and thin fibers can allow the fiber-cement composite to be applied by injection. In addition, such fibers can facilitate shear (flow) alignment during injection, thus result in tailorable mechanical properties of the resulting fiber-cement composite for patient-specific application.

Preferably, the average fiber length is less than 5.75 millimeter, most preferably less than 3.8 millimeter and ideally less than 1.9 millimeter. Such an average fiber length result in a bone cement according to the present invention with a good malleability and injectability.

In one aspect of the present invention the bone cement composite comprises a fiber volume fraction between 10 % and 18 %, preferably between 12 % and 16 %. In this range an optimal combination of mechanical strength and ductility can be obtained.

The base material of the bone cement composite (forming the composite's matrix, a term well known in the art) can be biodegradable or non-biodegradable. Biodegradable base material means that this material will be broken down in vivo and can be replaced over time by new growth of bone tissue, whereas non-biodegradable base material remains in the body unless surgically removed.

A biodegradable base material is preferably selected from the group consisting of magnesium calcium phosphate cements, calcium phosphate cements, brushite cements, hydroxyapatite cements, trimagnesium phosphate (Farringtonite) cements, amorphous magnesium phosphate cements, calcium sulphate cements and magnesium-ammonia cements. These cements' base materials have a good compatibility with the amorphous fibers according to the present invention.

A non-biodegradable base material is preferably selected from the group consisting of poly(methyl methacrylate), calcium aluminates and calcium silicates. Such a bone cement composite can be used for example for hip fracture prevention. Even in a non-biodegradable base material biodegradable magnesium-based or calcium-based additions such as fibers are beneficial by initially strengthening the resulting composite material and subsequently forming voids during *in vivo* degradation that foster bone-ingrowth. Additionally, leaking-out magnesium or calcium promotes bone growth and bone remodeling.

The bone cement composite according to the present invention can be used in the treatment of a wide variety of orthopedic conditions, in particular in the treatment of bone fractures, bone augmentation, regeneration of bone in large voids, or enhancement of primary fixation of biodegradable or nondegradable implants. By way of non-limiting example, the cement may be injected into the vertebral body for treatment of spinal fractures, injected into long-bone or flat-bone fractures to augment the fracture repair or to stabilize the fractured fragments, or injected into intact osteoporotic bones to improve strength. The cement is useful in the augmentation of a bone-screw or bone-implant interface. The bone cement may also be used to replace demineralized bone matrix materials. Because of its enhanced load-bearing capacity, the cement according to the present invention can provide scaffold support for various types of vertebral fracture and could be used in tibia plateau reconstruction, wrist fracture reconstruction, calcaneal reconstruction, spine reconstruction and prophylaxis strengthening of the hip bone.

Additionally, the bone cement composite according to the present invention is useful as bone filler in areas of the skeleton where bone is deficient. In this context, the cement is intended to fill, augment, and/or reconstruct maxillofacial osseous bone defects, preferably including periodontal, oral, and cranio-maxillofacial applications. The cements are packed gently into bony voids or gaps of the skeletal system (i.e., extremities, pelvis, and spine), including use in posterolateral spinal fusion or vertebral augmentation procedures with and without stabilizing hardware, such as pedicle screws and connection rods or expandable intra-vertebral implants. The bone cement may be used to fill defects which may be surgically created osseous defects or osseous defects created from traumatic injury to the bone. In certain embodiments, the cement provides a bone void filler that degrades and is replaced by bone during the healing process.

A further aspect of the present invention relates to the production of fibers. A preferred embodiment of an apparatus is disclosed below.

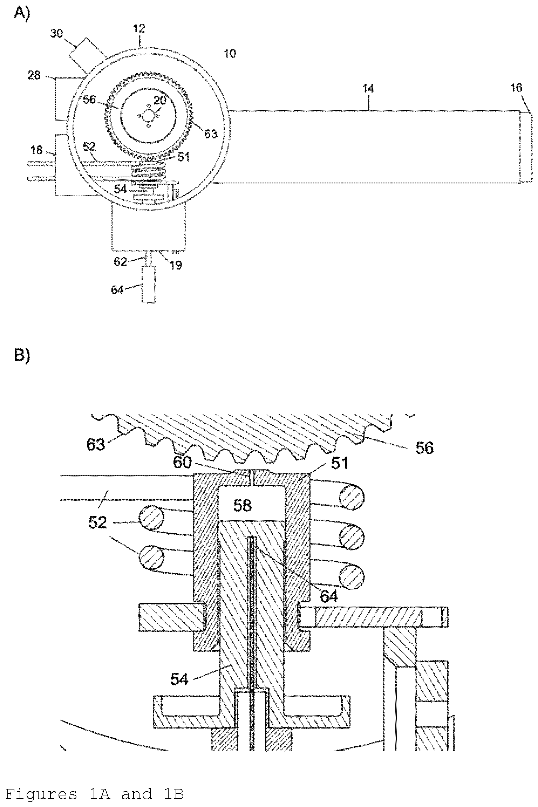

The metal alloy to be spun comprises at least three chemical elements in the alloy, wherein at least one of these elements is selected from the group consisting of magnesium and calcium. Said metal alloy is heated in a syringe feeding system (Figures 1A and 1B) by a heating device 52 until the metal alloy is fully molten, with the syringe feeding system comprising a cylinder 51 and a piston 54. Afterwards, the molten alloy is pressed through a nozzle 60 of the cylinder 51. Outside the nozzle, the melt forms a drop, in which a rotating spinning wheel 56 dips into and drags thin strands of molten alloy out from the drop. The strands of molten alloy being thereby in direct contact with the much colder rotating spinning wheel 56, which is made of metal, is subjected to a rapid cooling and solidification, resulting in fibers with a disordered, or amorphous, atomic-scale structure. The rotating spinning has preferably a sharpened external circumferential edge 63. Preferably, this edge is non-continuous, forming a plurality of teeth. Only the outermost, sharpened part of the sharpened external circumferential edge 63 dips into the molten alloy drop. Consequentially, the length of the produced fibers corresponds very precisely to the length of the part of the sharpened external circumferential edge that is part of such a tooth.

The apparatus preferably comprises a metallic chamber 10 having a cylindrical portion 12 and a tangentially extending collection tube 14 with a closable port 16 at the end remote from the cylindrical portion 12. A syringe feeding system comprising the cylinder 51 and the piston 54 (see Figures 1A and 1B) is mounted inside the chamber 10 right below a rotating spinning wheel 56 and heated by an inductive heating system 52. Necessary supply lines for a process gas such as argon, for the power of the heating system 52 and for the monitoring of control parameters such as the temperature of the melt exist. Power lines for the heating system 52 enter the chamber 10 via an electrical feedthrough 18. The chamber 10 can be evacuated by a vacuum pump via an evacuation stub 28 and can be supplied with a flow of an inert or reactive gas via a further feed stub 30. The wheel 56 is mounted on the inside of and axially parallel to the cylindrical portion 12 and is supported by bearings (not shown) on an axle 20 driven by an electric motor flanged to the rear of the cylindrical portion 12.

The piston 54 can be moved vertically with the help of the lifting rod 62 and a lifting motor 64 that is attached to it. The lifting rod 62 enters the chamber 10 via a linear feedthrough 19 that allows a vertical movement of the lifting rod 62 by at the same time maintaining different atmospheric conditions inside and outside the chamber 10. The lifting motor 64 can be equipped with a control system for control of the distance and velocity the rod moves and the force it can exert on the piston and thereby the melt.

The alloy to be spun is placed inside the melting cavity 58 that is formed by the assembled piston 54 and cylinder 51 and heated by a heating device 52. A temperature sensor 64, which is placed inside the piston 54 and right below the melting cavity 58 and the metal alloy to be processed, is used in interaction with the heating device 52 to control the temperature of the metal alloy. A piston 54 presses the molten alloy through the nozzle 60 of the cylinder 51 onto the rotating spinning wheel 56. The spinning wheel has preferably a sharpened external circumferential edge 63, which has a plurality of teeth. Such a wheel enables the convenient production of thin and short fibers. The nozzle 60 of the syringe feeding system comprising the cylinder 51 and the piston 54 has preferably a nozzle opening of circular shape. The diameter of the opening can be chosen within relatively wide limits, e.g., 0.5 millimeters to 10 millimeters to control the size of the molten alloy drop and the rate of flow of the molten alloy from the nozzle onto the sharpened external circumferential edge of the wheel in interaction with the lifting velocity of the piston 54.

A further aspect of the present invention relates to the coating of the fibers. Preferably, said fibers are partly or fully coated by a method selected from the group consisting of deposition coating, plasma electrolytic oxidation coating, plasma electrolytic anodization coating, anodization coating, fluoric conversion coating, $Mg(OH)_2$ coating, calcium phosphate conversion coating, phosphate conversion coating, hydroxyapatite coating, organic coating, biodegradable polymer coating, and sol-gel coating. This coating allows to prevent a chemical reaction between the fibers and the setting solution during setting of the cement. Furthermore, it can delay degradation onset during in vivo application or slow down degradation rate in the beginning of an in vivo application.

**Figures:**

Figure 1A and 1B show a schematic drawing of an apparatus for the fiber production. Figure 1B provides a cross-sectional view of the main components that are needed to form the molten alloy and control the fiber spinning process.

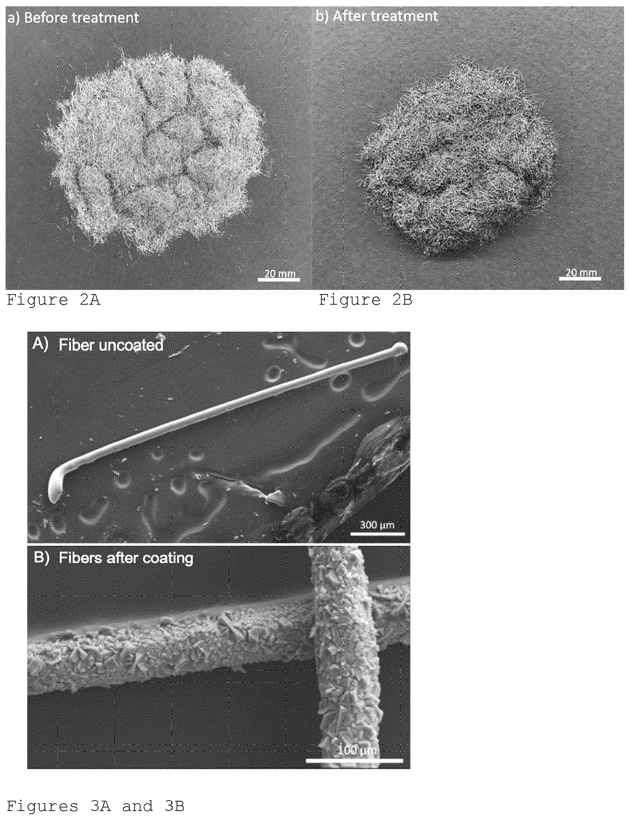

Figures 2A and 2B show the difference of the fibers before and after the treatment with diammonium hydrogen phosphate coating solution. A color change occurs due to the treatment.

Figures 3A and 3B show scanning electron microscopy images of an uncoated short fiber made from $Mg_{60}Zn_{35}Ca_5$ (in at %) (Figure 3A) and sections of fibers made from the same alloy after coating by immersion in diammonium hydrogen phosphate solution (Figure 3B).

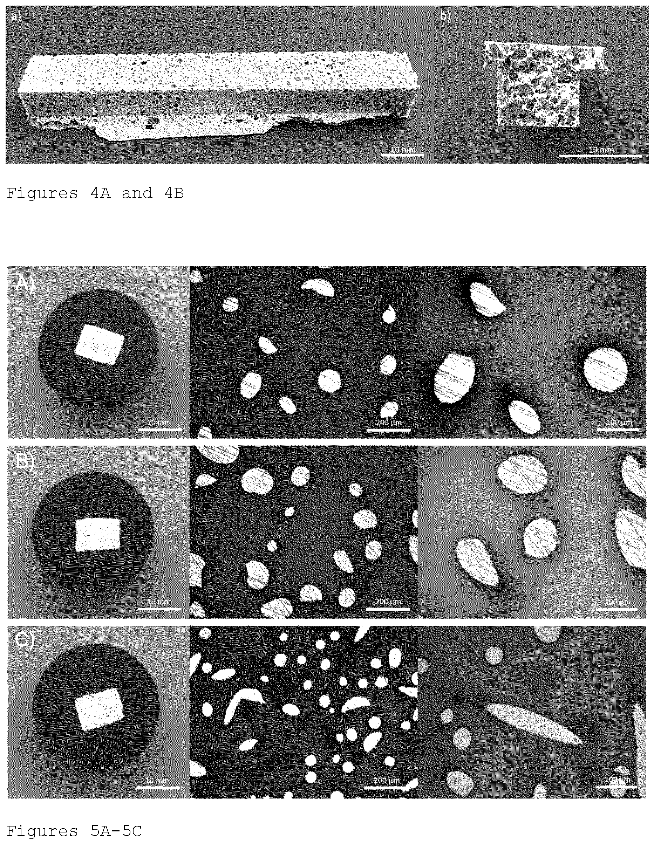

Figures 4A and 4B show an embodiment of the present invention with uncoated fibers of a fiber volume fraction of 10 % and a cement powder to liquid ratio of 2.5 grams per milliliter. The material's volume increased significantly during setting of the cement, and macropores formed on the surface as well as in the bulk.

Figures 5A-5C show an embodiment of the present invention with coated fibers and cement powder to liquid ratio of 3 grams per milliliter. The set fiber-reinforced bone cement was embedded in bakelite and ground with sandpaper, and the images were taken with a light microscope. The shown embodiments comprise coated fibers in a volume percentage of 10 % (Figure 5A), 15 % (Figure 5B) and 20 % (Figure 5C).

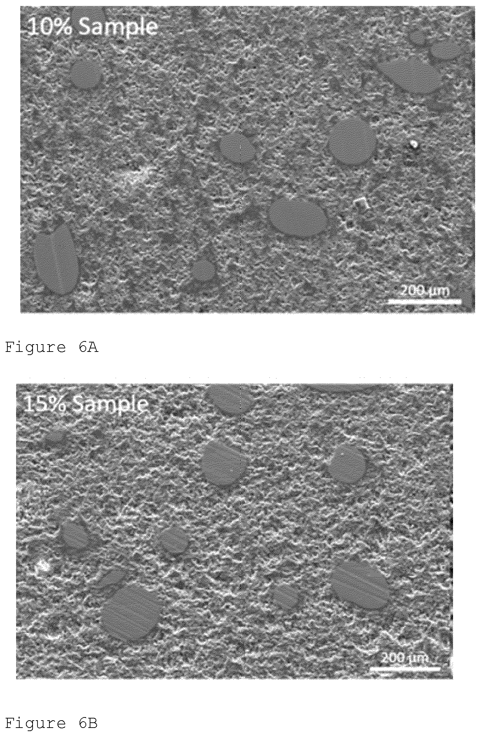

Figures 6A and 6B show scanning electron microscopy images of cross sections of embodiments that were produced using a cement powder to liquid ratio of 3 grams per milliliter and coated fibers in volume percentage of 10 % (Figure 6A) and 15 % (Figure 6B). The set fiber-reinforced bone cements were embedded in bakelite and ground with sandpaper before taking the images. The samples show no signs of pores.

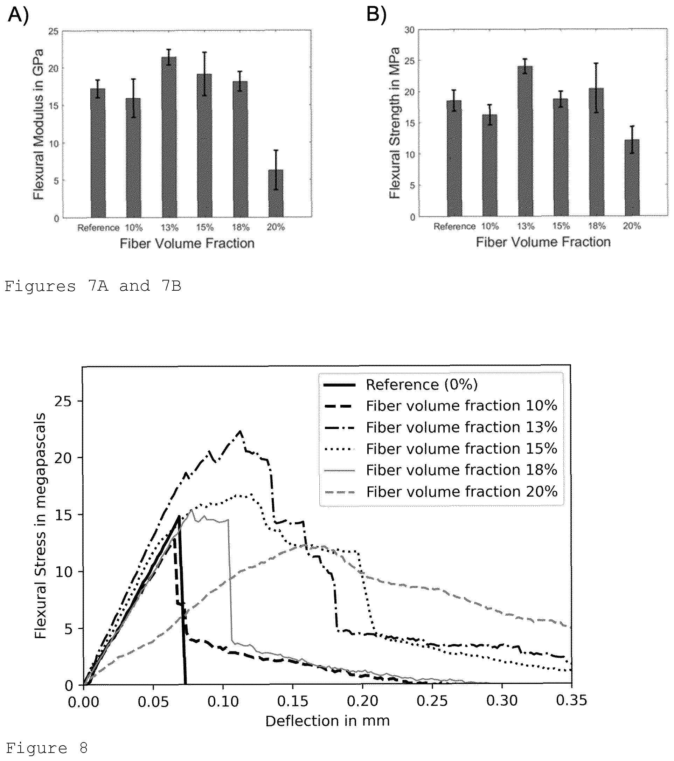

Figures 7A and 7B show graphical visualizations of determined mechanical properties of fiber-reinforced bone cements according to the present invention with respect to the fiber volume fraction. Coated fibers were used. The presented values are given as mean values with their respective standard deviation shown as error bars. "Reference" refers to plain bone cement without fiber addition.

Figure 8 shows typical graphs for bone cements according to the present invention with coated fibers of different fiber volume fractions as tested in 3-point bending. Additionally, plain bone cement without fiber addition was tested in the same way and is displayed labeled as "Reference".

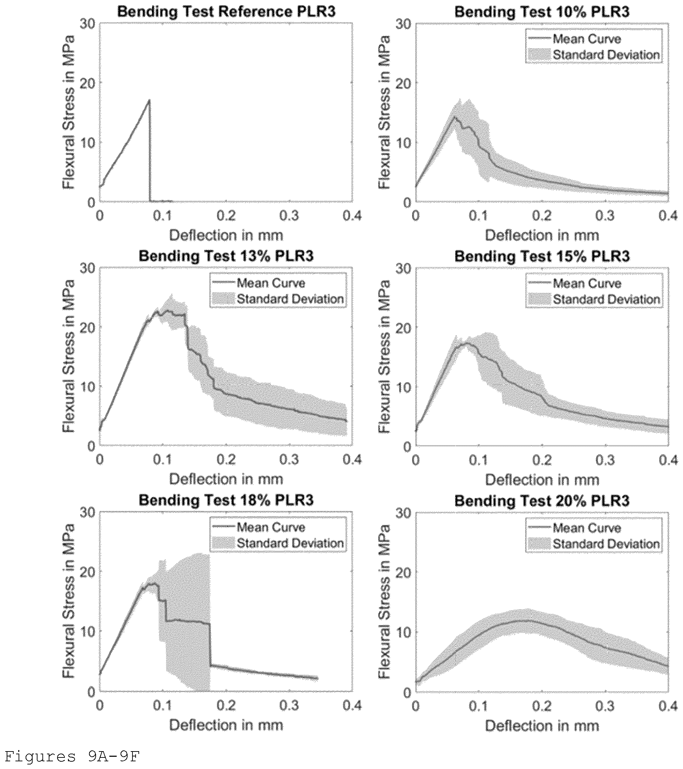

Figure 9A-9F shows mean curves (black) with the corresponding standard deviations (grey) of 3-point bending tests of bone cements without fibers (reference material) and with coated fibers of different fiber volume fraction. The reference material was chosen to be represented only by one typical curve, because the mean curve would indicate a false ductile behavior. All other samples do show ductile behavior.

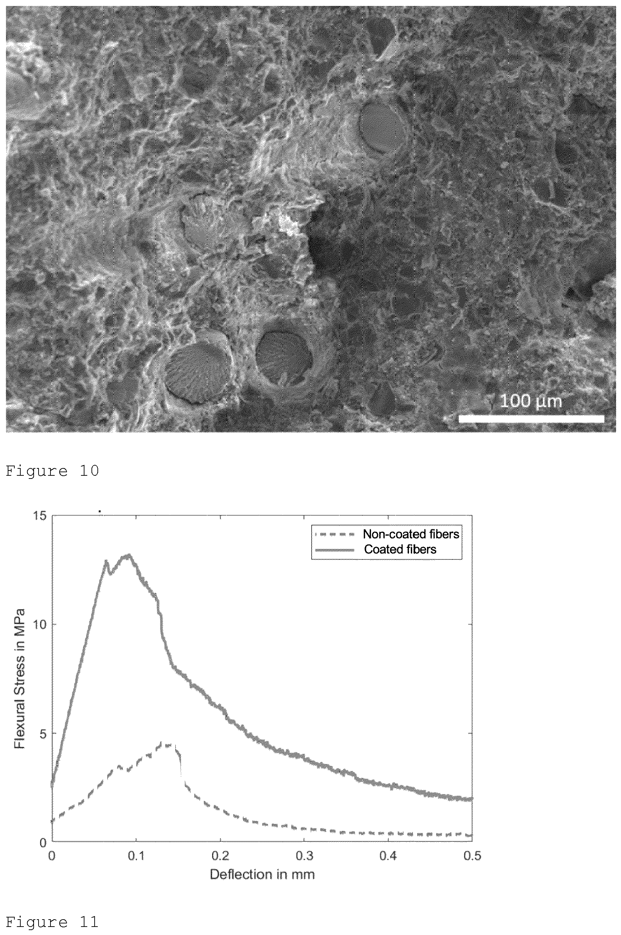

Figure 10 shows a scanning electron microscopy image of a typical fracture surface after a 3-point bending test of a fiber-reinforced bone cement composite according to the present invention. Several broken fibers are visible, in some cases with short pullout sections.

Figure 11 shows a comparison of fiber-reinforced bone cement composites according to the present invention with coated and non-coated fibers tested in 3-point bending. Both examples were prepared with a fiber volume fraction of 10 % and a powder to liquid ratio of 2.5 grams per milliliter. The example with uncoated fibers shows significant lower mechanical strength and a significant lower flexural modulus.

## Examples

## Fiber Production

An alloy with composition $Mg_{60}Zn_{35}Ca_5$, with the subscripted numbers giving the relative amounts of the respective element in atomic percent, made of high-purity components was used to produce highly amorphous fibers. This alloy has a density of about 3.062 grams per cubic centimeter and a melting temperature of about 390°C.

To produce the fibers, an in-house-built wire spinner was used. An original, simple version of this wire spinner has been reported in (B. Zberg, E. R. Arata, P. J. Uggowitzer, and J. F. Loffler, "Tensile properties of glassy MgZnCa wires and reliability analysis using Weibull statistics," Acta Materialia, vol. 57, no. 11, pp. 3223-3231, 2009, doi: 10.1016/j.actamat.2009.03.028) and was modified as described above and visualized in Figures 1A and 1B.

The amorphous structure of the fibers can be achieved by rapid cooling. This rapid cooling and solidification of the melt is carried out during the spinning process when the melt gets in contact with the spinning wheel. The above-mentioned spinning wheel construction and the small diameter of the fibers facilitate to reach the required high cooling rates. The maximum casting thickness for this alloy to achieve an amorphous state after solidification is around 2-4 mm, which is well above the targeted fiber thickness. This process allows to freeze the disordered state of the liquid alloy and hinders a long-range periodic arrangement of the metal atoms. The maximum casting thickness (also called critical casting thickness) is related with the minimum cooling rate (also called critical cooling rate) in rapid solidification according to the equation

$$\dot{T_c} = \frac{f}{d_c{}^2}$$

where $\dot{T_c}$ is the critical cooling rate in Kelvin per seconds, $f$ is a constant that has an empirically determined value ranging from about 20 to about 40 in centimeters squared times Kelvin per second, and $d_c$ is the critical casting diameter in centimeters. For the here employed alloy this equation results in a critical cooling rate of about 125 to 1000 Kelvin per seconds. To achieve the desired amorphous state the material needs to be solidified at a cooling rate that is higher than the critical cooling rate.

A block of the above-mentioned alloy ($Mg_{60}Zn_{35}Ca_5$, with the subscripted numbers giving the relative amounts of the respective element in atomic percent.) was cut into pieces of size 12 x 12 x 25 millimeters (around 10-12 grams of weight). After cutting, the pieces were ground until visible staining (potentially oxides) on the surface was mainly removed, and subsequentially cleaned with ethanol.

The wire spinning process was conducted inside of a high vacuum chamber. The small metal samples were placed on the piston and moved into the cylinder to the height of the heating system in the form of a copper induction coil. Afterwards, the vacuum pump was started, and the chamber was flushed with argon (400 millibars) two times starting at a pressure < $10^{-1}$ millibars. Before the chamber was ultimately filled with argon, a third time to 800 mbar, it was ensured with a high-vacuum pump that the pressure reached a value lower than $10^{-5}$ millibars. The sample was then molten with inductive heating of the water-cooled copper coil and temperature was measured with a thermocouple placed inside the piston. As soon as the desired temperature of about 430°C was reached, the molten sample was pushed out of the nozzle on top of the cylinder with the help of the motorized lifting rod that pushed the piston up. A small droplet of sample formed at the opening, before the rotating spinning wheel with a multitude of teeth started to extract the melt and eject the spun short fibers into the collection tube. Each tooth segment measured about 2 millimeters of the wheel's sharpened external edge. Thereby fibers of a length of about 2 millimeters and narrow length distribution were produced conveniently. The motorized lifting rod and the attached piston move upwards at a constant velocity and thereby provide a constant supply of molten material to the spinning wheel. At the end of the spinning process, the heating was stopped for the system to cool down. Subsequently, the fibers were extracted from the collection tube and stored in a plastic beaker. Afterwards, the fibers were sieved in a sieve shaker (Fritsch Laborgerätebau, W.-Germany) with a mesh size of 0.355 millimeters to get rid of all parts that are not fibers.

**Continuous fiber production**

In a similar manner, long, continues fibers were produced. However, instead of a spinning wheel with a multitude of teeth, a spinning wheel with a continuous sharpened external edge (without teeth) was used. The rotating speed of the spinning wheel was set to 1750 revolutions per minute.

**Fiber characterization:**

To verify the amorphous state of the fibers, x-ray diffraction measurements (X'Pert-Pro with Cu-K$\alpha$ source) in a $\Theta$-2$\Theta$ set up were performed. Each spectrum was recorded for 1 hour with a Soller slit. The measurements were recorded in an angle range of 5-70°. The x-ray diffraction measurements showed similar spectra for both the short and the long fibers. In both cases, no crystalline phase was detected, so it could be concluded that the fibers were amorphous. Furthermore, the geometry and surface quality were investigated and characterized by scanning electron microscopy (Hitachi SU-70) and light microscopy.

**Coating of the short fibers**

After the sieving, the short fibers were placed into a 3.5 molar diammonium hydrogen phosphate solution for 4 hours under constant stirring. After this treatment, the fibers were washed with deionized water several times until the waste water was not opaque anymore. Furthermore, the fibers were rinsed with ethanol three times and dried in air at room temperature.

As soon as the diammonium hydrogen phosphate solution was added to the fibers, many bubbles started to form originating from the fibers. The gas bubbles moved to the liquid's surface and an ammonium smell was present. Moreover, the solution turned opaque immediately after the fibers were added. Furthermore, during the treatment the color of these fibers changed from a shiny-silver to a mat-brownish color (Figures 2A and 2B).

An analysis of the coated fibers by scanning electron microscopy revealed that the surface experienced a significant change. The scanning electron microscopy images show that fibers after the immersion treatment feature a rough surface (Figure 3B) and not smooth as before the treatment (Figure 3A). The described treatment leads to coating of the fibers. This coating covers the entire length of the fibers as well as the face sides at each end of a fiber.

Chemical analysis performed by energy dispersive x-ray analysis of a coated fiber showed that the coating comprises mainly the elements magnesium, phosphorus, oxygen, calcium and zinc. Table 1 lists the elements detected in the fiber coating and its relative amounts in atomic percentages. Interestingly, the elements calcium and zinc are strongly underrepresented in the coating with respect to their relative amount in the fiber material.

Table 1: Chemical composition of the fiber coating as detected with energy dispersive x-ray analysis. The main elements detected are magnesium, phosphorus, and oxygen.

| Element | Atomic % |
|---|---|
| Magnesium | 19.45 |
| Phosphorus | 15.07 |
| Oxygen | 61.25 |
| Calcium | 0.93 |
| Zinc | 3.2 |

**Production of Fiber-reinforced Cements and Characterization**

**Bone cement preparation**

Composites of bone cement and fibers according to the present invention were produced with different fiber volume fractions. The cement material was produced as follows:

The individual components

- calcium hydrogen phosphate ($CaHPO_4$, >97%, Fluka Chemie AG, Buchs, Schweiz),
- calcium carbonate ($CaCO_3$, 98+% extra pure, Lot: 1879814, Fisher Chemical, Lough-borough, UK),
- magnesium hydrogen phosphate trihydrate ($MgHPO_4 \cdot 3H_2O$, 99%, Lot: 10227928, Alfa Aesar, Kandel, Germany) and
- magnesium hydroxide ($Mg(OH)_2$, >95 %, Fluka Chemie AG, Buchs, Schweiz)

were prepared in amounts according to Table 2 and filled together in a 375 milliliter aluminum oxide crucible. To ensure good mixing the powder mixture was thoroughly stirred with a spatula and slightly tapped on the ground for compacting. The crucible was then transferred into a furnace (Nabertherm Schweiz AG) and heated to 1100 °C with a ramping of 45 minutes. It was kept at that temperature for 5 hours before it was slowly cooled down to room temperature inside the furnace. After the resulting sintered powder cake had cooled down, it was crushed in an agate mortar to a fine powder and sieved in a sieve shaker (Fritsch Laborgerätebau, Germany) with a mesh size of 1 millimeter. Afterwards, the powder was dry-milled in a planetary ball mill (Changsha Tianchung Powder Technology Co. Ltd.) for 2 hours at 200 revolutions per minute. The beakers were 1 liter in volume and made of $ZrO_2$. Per beaker, 350 grams of zirconia balls of diameter 10 millimeter were added together with 125 grams of sieved powder. After ball milling, the powder was removed and stored in a dry place.

Table 2: Amounts of each cement component for one batch of bone cement as produced in the present example. The amount is given in mol.

| $CaHPO_4$ (mol) | $CaCO_3$ (mol) | $MgHPO_4 \cdot 3H_2O$ (mol) | $Mg(OH)_2$ (mol) |
|---|---|---|---|
| 0.5 | 0.25 | 1.5 | 0.75 |

A stock solution of a 3.5 molar diammonium hydrogen phosphate (($NH_4)_2HPO_4$, 99%, ACS reagent, Lot: A0421666, Acros Organics, Waltham, Massachusetts, USA) was prepared and used as the liquid part for the cement preparation and is required to achieve a setting of the cement. Therefore, it is also called setting solution.

Several samples were prepared with different ratios of cement powder to liquid setting solution. The ratio of cement powder to liquid setting solution in grams per milliliter is well known in the art as "powder to liquid ratio" (PLR).

Powder to liquid ratios in a range from 2.5 grams per milliliter to 4 grams per milliliter in steps of 0.5 grams per milliliter were realized. The samples with a powder to liquid ratio of 3 grams per milliliter were made by adding 9 grams of cement base material mixing with 3 milliliters of 3.5 molar diammonium hydrogen phosphate stock solution. The other samples were mixed according to their respective powder to liquid ratio. After mixing cement powder and stock solution, the mixture was immediately filled in a planetary mixer (Thinky Mixer ARE-250) without any grinding balls for 30 seconds at 2000 revolutions per minute. The resulting paste was then transferred into a silicon rubber mold with a plastic spatula. After the filling, the molds with the cement were slightly tapped on the table several times, to enhance the compaction. Each sample was individually prepared to ensure that all samples experience identical curing conditions with respect to time. For the curing process, a glass lid was put on the open surface of the mold to achieve a plane top surface. In a first phase, the samples were cured with the glass lid overnight. The next day, the glass lid was carefully removed, and the samples then continued the curing in air for another 24 hours. The whole curing process took 48 hours.

The crystalline phases of the cement after hardening were determined by x-ray powder diffraction (X'Pert-Pro with Cu-K$\alpha$ source) in a $\Theta$-$2\Theta$ set up. The spectrum was recorded for 1 hour with a Soller slit. The measurement was recoded in an angle range of 5-70°. To find the containing cement phases and their respective phase fractions, a Rietveld refinement was performed on the spectra. The following phases were detected: struvite ($NH_4MgPO_4 \cdot 6H_2O$), stanfieldite ($Ca_4Mg_5(PO_4)_6$), farringtonite ($Mg_3(PO_4)_2$), and brushite ($CaHPO_4 \cdot 2H_2O$) (Table 3) . However, the evidence for brushite is rather low and there is a chance that the detected values are artefacts.

Table 3: Crystalline cement phases and their respective phase fractions in percent as detected in the realized bone cements with respect to the powder to liquid ratio (PLR) in grams per milliliter. The error of these numbers is in the range of a few percentage points.

| Detected phase | Realized powder to liquid ratios | | | |
|---|---|---|---|---|
| | PLR 2.5 | PLR 3 | PLR 3.5 | PLR 4 |
| Struvite | 48.4 % | 35.8 % | 36.0 % | 30.5 % |
| Stanfieldite | 31.4 % | 43.4 % | 40.0 % | 43.1 % |
| Farringtonite | 14.9 % | 20.3 % | 21.7 % | 25.7 % |
| Brushite | 5.4 % | 0.5 % | 1.9 % | 0.7 % |

**Bone cement composite with uncoated fibers**

Fibers according to the present invention were prepared as described above by employing a melt spinning process. No modifications were made except of sieving. Then, the uncoated fibers were in a first step mixed with powder of magnesium calcium phosphate cement that was prepared as described above for 30 minutes in a planetary mixer (Thinky Mixer ARE-250) at 200 revolutions per minute, without grinding balls. Subsequently, in a second step, the resulting powder-fiber mixture was mixed with 3.5 molar diammonium hydrogen phosphate solution.

The resulting paste was then transferred into a silicon rubber mold with a plastic spatula. After the filling, the molds with the cement were slightly tapped on the table several times, to enhance the compaction. Each sample was individually prepared to ensure that all samples experience identical curing conditions with respect to time. For the curing process, a glass lid was put on the open surface of the mold to achieve a plane top surface. In a first phase, the samples were cured with the glass lid overnight. The next day, the glass lid was carefully removed, and the samples then continued the curing in air for another 24 hours. The whole curing process took 48 hours.

Samples of different powder to liquid ratio and different fiber volume fractions were produced (Table 4). All percentages in Table 4 are given in volume percent of fibers to cement base material.

Table 4: Number of samples produced with uncoated fibers with the corresponding powder to liquid ratio (PLR) and the realized fiber fractions in volume percent.

| PLR | Fiber fraction in volume percent | |
|---|---|---|
| | 10 % | 20 % |
| 2.5 | 1 | - |
| 3.5 | 1 | 1 |

After the resulting composite's setting process was finished, it was found that the composite significantly gained in volume, caused by large pores that formed inside the material (Figures 4A and 4B). By variation of the powder to liquid ratio and fiber volume fractions it was found that the pore formation was more pronounced with lower powder to liquid ratios and higher fiber volume fractions. It can be safely assumed that the large number of pores causes strongly reduced mechanical strength. Therefore, a structural application can be rather excluded as a potential field of application. However, for non- or low-loadbearing applications and some types of slow-degrading bone cements, for example apatitic calcium phosphate cements, the addition of such uncoated fibers and the resulting pores are beneficial to increase the degradation rate and/or bone-ingrowth of the resulting compound compared to the original bone cement without fibers. It is also conceivable to use instead of the here described highly amorphous magnesium-based fibers powder of magnesium or calcium or a magnesium-based alloy or a calcium-based alloy to achieve a similar pore-forming effect during setting of the cement.

**Bone cement composite with coated fibers**

Fibers according to the present invention were prepared as described above by employing a melt spinning process. Then these fibers were coated as described above by immersion in a 3.5 molar diammonium hydrogen phosphate solution. Subsequently, the coated fibers were mixed with magnesium calcium phosphate cement powder that was prepared as described above for 30 minutes in a planetary mixer (Thinky Mixer ARE-250) at 200 revolutions per minute, without grinding balls. After removing the power-fiber mixture and placing it in a glass beaker, 3.5 molar diammonium hydrogen phosphate solution was added.

The resulting paste was then transferred into a silicon rubber mold with a plastic spatula. After the filling, the molds with the cement were slightly tapped on the table several times, to enhance the compaction. Each sample was individually prepared to ensure that all samples experience identical curing conditions with respect to time. For the curing process, a glass lid was put on the open surface of the mold to achieve a plane top surface. In a first phase, the samples were cured with the glass lid overnight. The next day, the glass lid was carefully removed, and the samples then continued the curing in air for another 24 hours. The whole curing process took 48 hours.

Samples of different powder to liquid ratio and different fiber volume fractions were produced. Table 5 lists all the samples produced with the coated fibers.

Table 5: Number of samples produced with coated fibers with the corresponding powder to liquid ratio (PLR) and the realized fiber fractions in volume percent.

| PLR | Fiber fraction in volume percent | | | |
|-----|------|------|------|------|
|     | 10 % | 13 % | 15 % | 18 % |
| 2.5 | 1 | - | - | - |
| 3 | 6 | 3 | 6 | 3 |
| 3.5 | - | - | 2 | - |

Figure 5A to 5C show samples of the realized bone cement composites according to the present invention with coated fibers and a powder to liquid ratio of 3 grams per milliliter and with a fiber volume ratio of 10 % (Figure 5A), 15 % (Figure 5B), and 20 % (Figure 5C). The shown samples were embedded in bakelite and ground with 1200 and 4000 grid sandpaper. The images were taken with a light microscope.

Scanning electron microscopy images of these samples show furthermore excellent integration of the fibers into the cement matrix. No gaps or pores are visible in the samples with a fiber volume ratio of 10 % (Figure 6A) and a fiber volume ratio of 15 % (Figure 6B).

**3-Point Bending Tests**

Samples of fiber reinforced bone cements that were prepared as described above were tested in a universal testing machine (Schenck Trebel) in 3-point bending with a span length of 60 millimeters. Before the mechanical test, the setup was carefully aligned and fixed in this position. The samples had a dimension of about 75 x 10 x 7 millimeters. The surface that was covered with glass during the curing of the cement was placed facing down. This surface was clean, and no apparent defects were present. Therefore, it can be safely assumed that the result was not altered by stress concentrations from defects at the surface. The samples were tested immediately after the 48-hour curing period in dry condition and no further treatment. Testing was performed in speed-controlled mode at a testing speed of 0.5 millimeters per minute. Testing was continued until sample failure, or stopped when the force dropped below 10 newtons.

From the obtained data flexural strength and flexural modulus were calculated. The following formulas were

used.

Flexural strength:

$$\sigma = \frac{3F_{\mathrm{max}}L}{2wh^2}$$

Flexural modulus:

$$E_{\mathrm{flex}} = \frac{L^3 m}{4wh^3}$$

where $F_{\mathrm{max}}$ is the maximum recorded force, L is the span length, *w* is the width of the sample, h is the height of the sample and m is the gradient (i.e., slope) of the initial straight-line portion of the load deflection curve.

Table 6 presents the obtained results on mechanical properties of fiber-reinforced bone cement according to the present invention with respect to the fiber-volume ratio.

Table 6: Mechanical properties of tested fiber-reinforced bone cements as determined by a 3-point bending test. Fibers were coated in a process as described above. Flexural strength and flexural modulus are listed with respect to the fiber volume fraction. The values are stated as mean values and their standard deviation.

| Fiber volume fraction | Flexural strength in megapascals | Flexural Modulus in gigapascals |
|---|---|---|
| Reference (0 %) | 18.5 ± 1.7 | 17.1 ± 1.2 |
| 10 % | 16.2 ± 1.6 | 15.9 ± 2.5 |
| 13 % | 24.0 ± 1.4 | 21.3 ± 1.0 |
| 15 % | 18.7 ± 1.2 | 19.1 ± 2.9 |
| 18 % | 20.4 ± 3.9 | 18.1 ± 1.3 |
| 20 % | 12.1 ± 2.1 | 6.2 ± 2.6 |

It was found that the flexural modulus depends only moderately on the fiber volume fraction up to a fiber volume fraction of about 18 % (Figure 7A). The highest flexural modulus was obtained with 13 % of fiber volume fraction. Notable is that initially with 10 % fiber volume fraction the flexural modulus apparently decreased compared to the reference, which was plain bone cement without fibers. Moreover, at higher fiber volume fractions the flexural modulus drops significantly.

Furthermore, it was found that the addition of 10 % fibers apparently decreases the flexural strength compared to the reference material. However, at a fiber volume fraction of 13 % the composite shows a significant higher flexural strength compared to the reference material without fibers and to the composite with a fiber volume fraction of 10 % (Figure 7B). At this fiber volume fraction also the highest flexural strength of all investigated samples was found. A fiber volume fraction of 20 % led to a significant drop in flexural strength. These properties are visually represented as bar plots in Figures 7A and 7B.

Figure 8 shows typical 3-point bending graphs for all investigated specimens. The reference material (containing no fibers) was the only investigated material that failed in a clearly brittle manner. The graphs of the fiber-reinforced bone cements show occasionally occurring abrupt drops in stress, which can be attributed to single failing fibers.

Figures 9A-9F show the obtained results as mean curves in black and standard deviations in gray for all tested materials of different fiber volume fractions. In case of the reference material only one representative curve is depicted since the mean curve would indicate a ductile failure which was never observed. Only the reference samples with no fibers clearly showed brittle failure. All other samples showed ductile failing behavior. All investigated specimens failed by showing only one visible crack. Multiple crack formation was not observed.

Figure 10 shows a scanning electron microscopy image of a typical fracture surface after the 3-point bending test of fiber-reinforced bone cement. The analysis of the fracture surface revealed that most of the fibers broke directly at the fracture surface, and only few appeared to be pulled out. This indicates an excellent fiber-cement interface strength and furthermore leads to the conclusion that even shorter fibers of the same diameter would lead to equally good mechanical properties. Shorter fibers would further improve malleability and injectability of the fiber-reinforced bone cement during application.

A comparison of the fiber-reinforced bone cements with coated and uncoated fibers shows a significant difference in their mechanical behavior (Figure 11). Fiber-reinforced bone cements with uncoated fibers exhibit a significantly lower flexural strength and a lower flexural modulus compared to fiber-reinforced bone cements with coated fibers. On the positive side, fiber-reinforced bone cements with uncoated fibers also show a ductile failing behavior, although not as pronounced as fiber-reinforced bone cements with coated fibers.

**Injectability Study**

Bone cement composites according to the present invention with coated fibers with different powder to liquid ratios (PLR) were tested on their injectability. The bone cement composites were prepared as described above. Recorded was the consistency of the paste after mixing with the setting solution, how big the minimum syringe opening was at which the paste was still injectable, how well it was injectable with this opening, if the push-out force increases during the injection, and if glycerol would enhance the injectability if it was used instead of stock solution. All tests were done by hand and qualitatively assessed rather than quantitatively.

It was found that the injectability improved significantly with the addition of glycerol to the mixture of powder and diammonium hydrogen phosphate solution. With this combination the material was extrudable with no force increase and no separation of liquid and powder phases. It is equally conceivable that other liquid additions of high viscosity can improve injectability in a similar manner.

**Claims**

1. Bone cement composite comprising at least one cement base material and biodegradable metallic fibers made of a metal alloy, said fibers comprising at least three chemical elements in the alloy, wherein at least one of these elements is selected from the group consisting of magnesium and calcium, **characterized in that** the fibers are amorphous in a volume fraction of more than 75%, with the remaining part of the fibers being of crystalline atomic structure.

2. Bone cement composite according to any of the preceding claims, **characterized in that** the fibers are amorphous in a volume fraction of more than 90%, or more preferable in a volume fraction of 95%, or particularly preferable in a volume fraction of more than 98%, with the remaining part of the fibers being of crystalline atomic structure.

3. Bone cement composite according to any of the preceding claims, **characterized in that** the fibers are at least partly, preferably completely coated with a biodegradable protective layer, wherein said protective layer preferably comprises salts, oxides, biodegradable polymers or small biocompatible organic molecules or a mixture thereof.

4. Bone cement composite according to any of the preceding claims, wherein the fibers comprise at least 45 at% calcium.

5. Bone cement composite according to any of the preceding claims, wherein the fibers comprise between 45 at% and 80 at% of calcium, between 5 at% and 30 at% of magnesium and between 2 at% and 35 at% of zinc.

6. Bone cement composite according to claims 1 to 3, wherein the fibers comprise at least 55 at% magnesium.

7. Bone cement composite according to claim 6, wherein the fibers comprise between 55 at% and 80 at% of magnesium, between 15 at% and 40 at% of zinc and between 1 at% and 10 at% of calcium, preferably between 60 at% and 75 at% of magnesium, between 20 at% and 35 at% of zinc and between 2 at% and 7 at% of calcium.

8. Bone cement composite according to any of the preceding claims, wherein the fibers comprise magnesium and/or calcium and one or more elements selected from the group consisting of copper, nickel, zinc, palladium, and rare-earth elements, preferably magnesium and calcium and one or more elements selected from the group consisting of copper, nickel, zinc and palladium.

9. Bone cement composite according to any of the preceding claims, wherein the fiber has a diameter that is smaller

than 150 μm, preferably smaller than 100 μm and most preferably smaller than 50 μm.

10. Bone cement composite according to any of the preceding claims, wherein the bone cement composite comprises a fiber volume fraction between 10 % and 18 %, preferably between 12 % and 16 %.

11. Bone cement composite according to any of the preceding claims, wherein the bone cement base material is selected from the group consisting of magnesium calcium phosphate cements, calcium phosphate cements, brushite cements, hydroxyapatite cements, trimagnesium phosphate (Farringtonite) cements, amorphous magnesium phosphate cements, calcium sulphate cements and magnesium-ammonia cements.

12. Bone cement composite according to any of the preceding claims for use in the treatment of bone fractures, bone augmentation, regeneration of bone in large voids, or enhancement of primary fixation of biodegradable or nondegradable implants, preferably for use of posterolateral spinal fusion or vertebral augmentation procedures with stabilizing hardware.

13. Bone cement composite according to claim 12 for use as bone filler in areas of the skeleton where bone is deficient to fill, augment, and/or reconstruct maxillofacial osseous bone defects, preferably including periodontal, oral, cranio-maxillofacial and vertebral applications.

14. Fibers made of a metal alloy, said fibers comprising at least three chemical elements as alloying elements, wherein at least one of these elements is selected from the group consisting of magnesium and calcium, **characterized in that** the fibers are amorphous in a volume fraction of more than 75%, with the remaining part of the fibers being of crystalline atomic structure, **characterized in that** the fibers are at least partly, preferably completely coated with a biodegradable protective layer.

15. Fibers according to claim 14, wherein the fibers are coated by a biodegradable protective layer, wherein the protective layer comprises salts, in particular phosphate salts or fluoride salts, oxides, biodegradable polymers or small biocompatible organic molecules or a mixture thereof.

A)

B)

Figures 1A and 1B

Figure 2A

Figure 2B

Figures 3A and 3B

Figures 4A and 4B

Figures 5A-5C

Figure 6A

Figure 6B

A)

B)

Figures 7A and 7B

Figure 8

Figures 9A-9F

Figure 10

Figure 11

Europäisches
Patentamt

European
Patent Office

Office européen
des brevets

# EUROPEAN SEARCH REPORT

Application Number

EP 22 19 1064

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | CN 111 773 432 A (INST METAL RESEARCH CAS) 16 October 2020 (2020-10-16) | 1,2,4-13 | INV. A61L27/04 |
| Y | * page 2, paragraphs 0005, 0009, 0010, 0012 * | 3,14,15 | A61L27/42 A61L27/58 |
| Y | US 2013/150978 A1 (NIES BERTHOLD [DE] ET AL) 13 June 2013 (2013-06-13) * claims 11, 16, 17 * * page 3, paragraphs 0027, 0028 * | 3,14,15 | |

TECHNICAL FIELDS
SEARCHED      (IPC)

A61L

The present search report has been drawn up for all claims

| Place of search | Date of completion of the search | Examiner |
|---|---|---|
| The Hague | 31 January 2023 | Dudás, Eszter |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons

...................................................................................................

& : member of the same patent family, corresponding
   document

EPO FORM 1503 03.82 (P04C01)

## ANNEX TO THE EUROPEAN SEARCH REPORT
## ON EUROPEAN PATENT APPLICATION NO.

EP 22 19 1064

This annex lists the patent family members relating to the patent documents cited in the above-mentioned European search report.
The members are as contained in the European Patent Office EDP file on
The European Patent Office is in no way liable for these particulars which are merely given for the purpose of information.

31-01-2023

| Patent document cited in search report | | Publication date | Patent family member(s) | | Publication date |
|---|---|---|---|---|---|
| CN 111773432 | A | 16-10-2020 | NONE | | |
| US 2013150978 | A1 | 13-06-2013 | EP | 2582407 A1 | 24-04-2013 |
| | | | US | 2013150978 A1 | 13-06-2013 |
| | | | WO | 2011157758 A1 | 22-12-2011 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20140007986 A1
- CN 106273680 A
- US 5543187 A

**Non-patent literature cited in the description**

- **I. LODOSO-TORRECILLA ; J.J.J.P. VAN DEN BEUCKEN ; J.A. JANSEN.** Calcium phosphate cements: Optimization toward biodegradability. *Acta Biomater.,* 2021, vol. 119, 1-12
- **R. KRÜGER ; J.M. SEITZ ; A. EWALD ; F.W. BACH ; J. GROLL.** Strong and tough magnesium wire reinforced phosphate cement composites for load-bearing bone replacement. *J Mech Behav Biomed Mater,* 2013, vol. 20 (C), 36-44
- **B. ZBERG ; E. R. ARATA ; P. J. UGGOWITZER ; J. F. LOFFLER.** Tensile properties of glassy MgZnCa wires and reliability analysis using Weibull statistics. *Acta Materialia,* 2009, vol. 57 (11), 3223-3231